# DEMANDE DE BREVET EUROPEEN

(11) **EP 1 321 125 A1**
(43) Date de publication de la demande: **25.06.2003**
(21) Numéro de dépôt: 02292957.4
(22) Date de dépôt: 29.11.2002
(51) Int. Cl.: A61K 7/06

(54) **Composition cosmétique comprenant un polymère à squelette non siliconé et à fonctions réactives**

(30) Priorité: 18.12.2001 FR 0116384
(71) Demandeur: L'Oreal, 92585 Clichy Cédex (FR)
(72) Inventeur: Samain, Henri, 91570 Bievres (FR); Rollat-Corvol, Isabelle, 75017 Paris (FR); Giroud, Franck, 92110 Clichy (FR); Mougin, Nathalie, 75011 Paris (FR); Livoreil, Aude, 75006 Paris (FR)
(74) Mandataire: Bourdeau, Françoise

(57) **Abrégé**

L'invention a pour objet des compositions cosmétiques, notamment capillaires, comprenant au moins un polymère à squelette non siliconé, comprenant des fonctions chimiques réactives, apte à former un gainage rigide sur les cheveux. Elle vise également un procédé cosmétique comprenant l'application de cette composition sur les cheveux ainsi que son utilisation pour réaliser un gainage rigide sur les cheveux.

## Description

L'invention a pour objet des compositions cosmétiques non tackantes, notamment capillaires, comprenant au moins un polymère à squelette non siliconé, comprenant des fonctions chimiques réactives, apte à former un gainage rigide sur les cheveux. Elle vise également un procédé cosmétique comprenant l'application de cette composition non tackante sur les cheveux ainsi que son utilisation pour réaliser un gainage rigide sur les cheveux.

Conformément à l'invention, pour déterminer si les fonctions organiques libres (F) d'un polymère (P) constituent des fonctions chimiques réactives, on met en oeuvre le test 1 décrit ci après :
(1) On réalise une solution ou une dispersion du polymère (P) dans un solvant cosmétiquement acceptable choisi parmi l'eau, les alcools en C, à C₄, les esters, les cétones et de préférence l'eau, cette solution ou dispersion ayant une teneur relative en polymère comprise entre 0,1 et 50 % en poids ;
(2) Pendant une durée comprise entre 1 à 60 minutes, on laisse la solution ou la dispersion de polymère (P) au repos ou bien on la soumet à l'une au moins des opérations suivantes :
   (i) On l'agite;
   (ii) On l'active par une température comprise entre 0°C et 100°C ;
   (iii) On l'active par un pH compris entre 1 et 13 ;
   (iv) On l'active par au moins un additif chimique (A) choisi parmi les molécules ou les polymères portant des fonctions chimiques libres susceptibles de réagir avec au moins une fonction organique libre (F) du polymère (P), l'additif (A) étant, de préférence, un polymère portant des fonctions chimiques identiques à celles des cheveux, choisies parmi les fonctions aminé, alcool, acide carboxylique, disulfure et thiol ;
(3) On examine la solution ou la dispersion de polymères (P), par des méthodes connues de l'homme du métier, en particulier par spectrométrie infrarouge ou RAMAN, afin de déterminer si au moins une fonction organique libre (F) du polymère (P) a donné naissance à la formation de liaisons covalentes, reliant, par exemple :
   - deux atomes présents dans des fonctions organiques libres (F) appartenant à différents polymères (P),
   - un atome présent dans le polymère (P) et un atome présent dans 1' additif chimique (A) ;
*(4)* Le polymère (P) est qualifié de *« polymère à fonctions réactives »* si la formation de liaison(s) covalente(s) est (sont) détectée(s) au point (3) et à la condition que celle(s)-ci ne résulte(nt) pas exclusivement d'une hydrolyse ou d'une oxydation de ce dernier.

Les polymères à squelette non siliconé et à fonctions réactives conformes à la présente invention présentent la particularité d'être capables de former des liaisons covalentes par la mise en oeuvre du test 1. Cette caractéristique les distingue de la plupart des polymères connus dans le domaine des compositions capillaires, lesquels ne réagissent pas, dans les conditions du test 1, en formant des liaisons fortes, mais tout au plus en interagissant entre eux ou avec des additifs par des liaisons, de type liaison hydrogène ou saline.

La présente invention exclut les polymères à squelette non siliconé à fonctions réactives photoactivables, c'est-à-dire les polymères comportant des fonctions chimiques, qui irradiées à une longueur d'onde comprise entre 200 et 800 nm, donnent naissance, en une ou plusieurs étapes, à la formation de nouvelles liaisons covalentes.

On entend par *« polymère à squelette non siliconé »,* un polymère n'étant pas exclusivement constitués d'enchaînement - Si-O-Si-, dans sa chaîne principale.

Les produits cosmétiques destinés aux traitements des cheveux emploient souvent des polymères. Ils permettent d'obtenir des effets de maintien de la forme de la coiffure, des effets de douceur ou des effets de brillance, par exemple.

Certaines des compositions utilisant les polymères présentent des inconvénients pouvant être gênants. Par exemple, si après avoir appliqué un produit contenant des polymères, une personne passe la main sur ses cheveux, une partie des polymères peut, au moment du contact, se déposer sur ses doigts. Ce phénomène de transfert, même s'il est partiel, laisse une impression de cheveux sales ou collants. L'ampleur de ce transfert peut dépendre des conditions climatiques. Ainsi, en milieu humide, il est souvent spécialement important.

Par ailleurs, lorsque du sébum recouvre les cheveux, soit sur leur longueur, soit à la racine, et qu'on applique sur ceux-ci un produit cosmétique, comme un produit coiffant, ce dernier peut non seulement être inefficace, mais, de surcroît, rendre les cheveux encore plus artificiellement brillants et plus sales.

Un autre inconvénient des polymères utilisés en cosmétique réside dans le fait que ceux-ci dessèchent parfois les cheveux, causant ainsi une altération de leur toucher et une diminution de l'effet escompté du produit, par exemple l'effet de fixation et/ou de maintien de la coiffure. On peut encore citer, comme inconvénient supplémentaire, le fait que les polymères déposés sur les cheveux soient très vite éliminés lors des shampooings.

En particulier, lorsqu'on choisit d'utiliser des polymères formant sur les cheveux un revêtement, notamment un film, de consistance rigide, le toucher des cheveux est souvent spécialement rêche ou collant, et désagréable. En outre, ce revêtement rigide s'élimine immédiatement lors du lavage des cheveux, et il est donc nécessaire de réappliquer du produit, au moins après chaque shampooing.

Il existe donc un besoin de réaliser des compositions cosmétiques non tackantes qui soient améliorées par rapport aux compositions de l'art antérieur, et en particulier, qui ne collent pas aux doigts après application sur les cheveux, ne les dessèchent pas et leur donnent de bonnes propriétés cosmétiques, même en présence de sébum et qui soient rémanente, face aux lavages répétés.

De manière surprenante et inattendue, la Demanderesse a découvert qu'il était possible d'atteindre les objectifs énumérés ci-dessus en sélectionnant les polymères introduits dans les compositions cosmétiques non tackantes, selon la nature des fonctions chimiques qu'ils portent et selon les caractéristiques du film qu'ils forment sur les cheveux.

L'invention a donc pour objet une composition cosmétique non tackante, notamment capillaire, comprenant, dans un milieu cosmétiquement acceptable, au moins un polymère à squelette non siliconé, comprenant au moins deux fonctions chimiques réactives, identiques ou différentes, non photoactivables, caractérisée par le fait que:
(i) un film obtenu par séchage de la composition à température ambiante (22 ± 2°C) et à un taux d'humidité relative de 50 % ± 5%, présente un module d'Young compris entre 100 et 2000 MPa, bornes incluses, mesuré à une épaisseur de 0,5 mm et à une traction de 20 mm/min,
(ii) la ou les fonctions réactives est (sont) choisie(s) parmi les groupements monovalents ou divalents ci-après :
   - époxy,
   - anhydride,
   - chlorure d'acide,
   - éthylène-imino,
   - aldéhyde,
   - acétal ou hémi-acétal,
   - aminal ou hémi-aminal,
   - cétone, alpha-halocétone ou alpha-hydroxycétone,
   - lactone ou thiolactone,
   - isocyanate,
   - thiocyanate,
   - ester de N-hydroxysuccinimide,
   - imide,
   - imine,
   - imidate,
   - oxazoline, oxazolinium, oxazine ou oxazinium,
   - pyridyl-thio,
   - thiosulfate,
   - acétoalkylate répondant à la formule : -OCO-A'-COCH₃,
      A' représentant une liaison, un groupement alkylène, linéaire ou ramifiée, comprenant de 1 à 5 atomes de carbone,
   - AX,
   - ASO₂X,
      . X est un groupe partant choisi parmi les halogènes, OSO₃H, SO₂CH3, SO₂C₂H₅, SO₂Tos, N(CH3)₃, OPO₃R₂, CN,
         Tos représentant un groupement tosylate,
         R représentant soit un atome d'hydrogène, soit un radical alkyle en C₁ à C₅,
      . A est un groupe alkylène, arylène, alralkylène comportant entre 1 et 22 atomes de carbone, éventuellement interrompu par un ou plusieurs cycles insaturés, et comportant, éventuellement, un ou plusieurs hétéroatomes, tels que N, S, O,
(iii) le polymère à squelette non siliconé, comprenant au moins deux fonctions chimiques réactives étant autre que le copolymère vinylbutyral/vinylalcool/vinylacétate.

Les « *compositions tackantes »,* à savoir les compositions qui donnent après application sur les fibres kératiniques et séchage, un matériau coiffant présentant un profil de décollement défini par au moins :
- une force maximale de décollement Fₘₐₓ > 1 Newton, et
- de préférence, en outre, par une énergie de séparation E_{s(M/V)} du matériau mis en contact avec une surface en verre, inférieure à 300 µJ,
Fₘₐₓ et E_{s(M/V)} étant mesurés selon le protocole décrit dans la demande internationale WO98/38969, déposée par la Demanderesse, sont exclues de la présente demande.

Un autre objet de la présente invention concerne un procédé cosmétique comprenant l'application de cette composition non tackante.

Encore un autre objet de la présente invention concerne l'utilisation de cette composition non tackante, pour réaliser un gainage rigide sur les cheveux.

Au sens de la présente invention, on entend par *« gainage »,* une enveloppe formée à la surface de chaque cheveu, après séchage de la composition cosmétique non tackante. Cette enveloppe a quasiment la forme d'un cylindre creux qui peut s'étendre de la racine jusqu'à la pointe des cheveux et qui adhère fortement à ceux-ci.

Sans être liée par une quelconque théorie, la Demanderesse pense que les polymères, à squelette non siliconé, présents dans les compositions cosmétiques selon l'invention peuvent, du fait de leurs fonctions réactives, identiques ou différentes, réagir totalement ou partiellement, soit sur eux-mêmes, soit entre eux, soit avec les cheveux, sensibilisés ou non, soit avec un ou plusieurs constituants réactifs de la composition capillaire et ceci, après application de la composition cosmétique sur les cheveux, pour former un gainage. Le mécanisme de la formation du gainage pourra être mieux compris au moyen des exemples schémas de réaction ci-après :
1) Réaction de deux polymères à fonctions réactives époxy avec un ingrédient réactif de la composition ayant pour formule RHN-A-NHR', 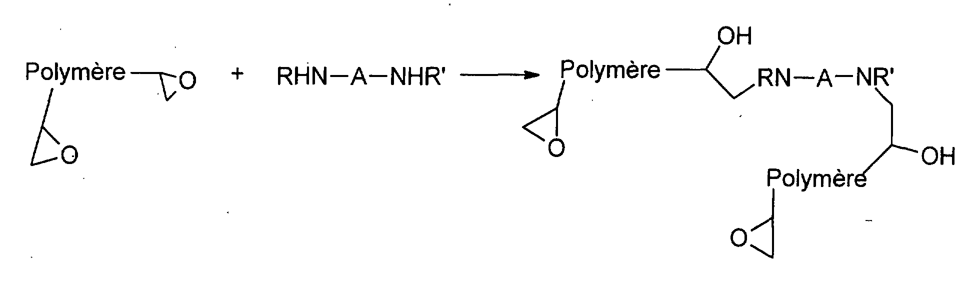
2) Réaction de deux polymères à fonctions réactives époxy avec une fonction amine du cheveu,

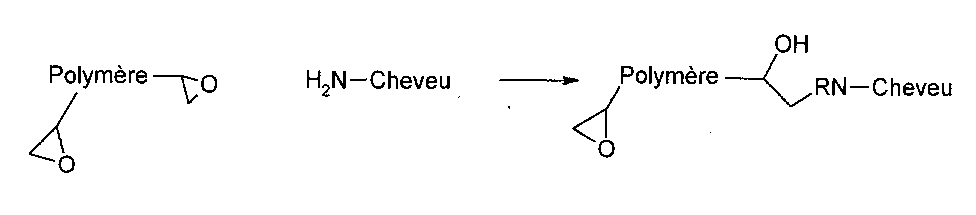

La réaction des polymères à squelette non siliconé selon l'invention entre eux et/ou avec les cheveux peut être favorisée par apport de chaleur, par ajout des constituants, par exemple, des agents régulateurs de pH, des actifs chimiques, comme des oxydants, des réducteurs, des inhibiteurs ou des catalyseurs de polymérisation.

De préférence, le polymère à squelette non siliconé, contenant au moins deux fonctions réactives contient moins de 50 % en nombre de fonctions ester d'acide carboxylique par rapport au nombre total des fonctions chimiques réactives.

Avantageusement, le groupe partant X représente un halogène choisi parmi le brome, le chlore, l'iode et le fluor.

Lorsque le polymère à squelette non siliconé, ayant au moins deux fonctions réactives contient un groupement époxy, ce dernier est, de préférence, monovalent et répond à la formule (I) : 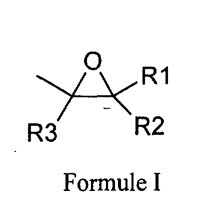 dans laquelle R ₁ , R ₂ et R ₃ représentent, indépendamment les uns des autres :
- un atome d'hydrogène,
- un groupement alkyle, linéaire ou ramifié, comprenant de 1 à 20 atomes de carbone, éventuellement interrompu par un ou plusieurs hétéroatome(s) choisi parmi O, N, S, Si et F, et éventuellement substitué par un ou plusieurs radicaux hydroxy ou amino,
- un groupement aryle comprenant de 6 à 22 atomes de carbone, ou
- un groupement aralkyle, le groupement alkyle comprenant de 1 à 20 atomes de carbone,
- un hétérocycle de 5 à 7 chaînons.

Lorsque le polymère à squelette non siliconé, ayant au moins deux fonctions réactives contient un groupement anhydride d'acide carboxylique, ce dernier est, de préférence, monovalent et répond :
(a) soit à la formule (II) : 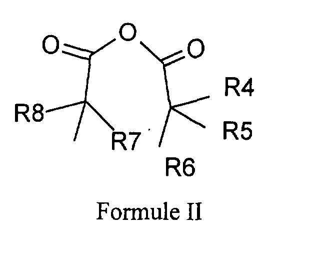 dans laquelle R ₄ , R ₅ , R ₆ , R ₇ et R ₈ ont, indépendamment les uns des autres, les mêmes significations que celles données pour R₁, R ₂ et R ₃ dans la formule (I),
(b) soit à la formule (III): 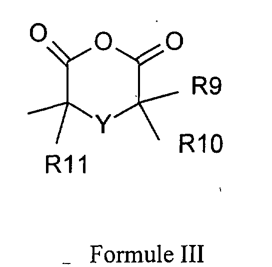
dans laquelle Y représente :
- une liaison,
- un hétéroatome choisi parmi O, N, S, Si et F,
- un radical alkyle ou alkylène, substitué ou non par un ou plusieurs radicaux hydroxy ou amino, contenant entre 1 et 5 atomes de carbone,
- un radical ralkylène, contenant entre 7 et 10 atomes de carbone ou
- un radical polydiméthylsiloxane contenant entre 1 et 6 atomes de silicium, et
   R₉, R₁₀ et R ₁₁ ont, indépendamment les uns des autres, les mêmes significations que celles données pour R₁, R ₂ et R ₃ dans la formule (I).

Lorsque le polymère, à squelette non siliconé ayant au moins deux fonctions réactives contient un groupement acétoalkylate, ce dernier est, de préférence, inclus dans un groupe répondant à la formule (IV) :

-R'₁-OCO-A'-COCH₃ Formule IV

dans laquelle R'₁ est obtenu par élimination de l'un quelconque des atomes d'hydrogène du radical R₁ défini dans la formule (I) et A' a la signification indiquée ci-avant.

Lorsque le polymère à squelette non siliconé, ayant au moins deux fonctions réactives contient un groupement chlorure d'acide, ce dernier est, de préférence, inclus dans un groupe répondant à la formule (V) :

-R'₁-COCl Formule V

dans laquelle R'₁ a la même signification que dans la formule (IV).

Lorsque le polymère à squelette non siliconé, ayant au moins deux fonctions réactives contient un groupement isocyanate, ce dernier est, de préférence, inclus dans un groupe répondant à la formule (VI) :

-R'₁-NCO Formule VI

dans laquelle R'₁ a la même signification que dans la formule (IV).

Lorsque le polymère à squelette non siliconé, ayant au moins deux fonctions réactives contient un groupement acétal, ce dernier est, de préférence, monovalent et inclus dans l'une au moins des formules (VII), (VIII) ou (IX) : 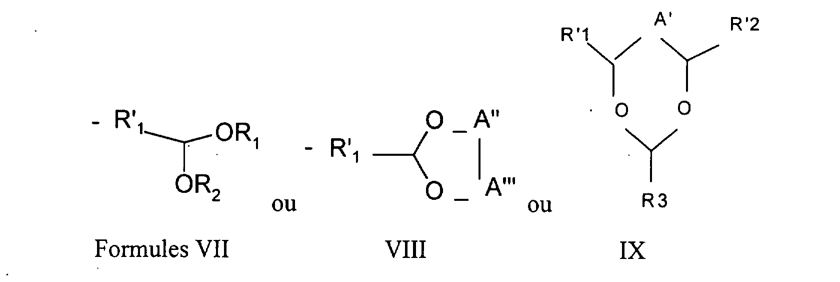 dans laquelle :
R ₁ , R ₂ et R ₃ ont la signification indiquée pour la formule (I),
R'₁ et R'₂ sont obtenus par élimination de l'un quelconque des atomes d'hydrogène du radical R₁ ou R₂ défini dans la formule (I)
A" et A"' sont identiques ou différents et représentent un groupement alkyle ou alkylène, linéaire ou ramifiée, comprenant 1 à 5 atomes de carbone, éventuellement interrompu par un ou plusieurs hétéroatome(s) choisi parmi O, N, S, Si, F, et éventuellement substitué par un ou plusieurs radicaux hydroxy ou amino.

On préfère, en particulier, en tant que polymères à squelette non siliconé contenant au moins deux fonctions réactives, les polymères choisis parmi :
(a) les copolymères synthétisés à partir de monomères (méth)acrylate et acrylate, à fonctions acétoacétate, répondant à la formule générale (IV.1): 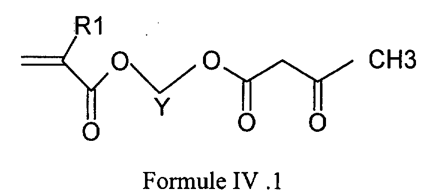 dans laquelle R₁ représente H ou CH₃ et Y a la même signification que celle indiquée pour la formule (III), R₁ représentant, de préférence, CH₃ et Y représentant, de préférence, - (CH₂ )₂.
(b) les polymères synthétisés à partir de monomères (méth)acrylate et (méth)acrylamide à fonctions acétales, ces monomères répondant aux formules générales (VII. 1) ou (VIII.1): 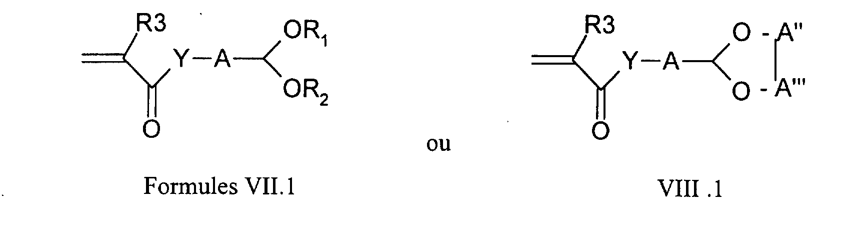
   avec Y = O ou NH
   R₃ = H ou CH₃
   A, A", A"', R₁ et R₂ ayant les mêmes significations que précédemment.
   Les copolymères synthétisés à partir de N-éthyl acétal acrylamide de formule (VII.2) 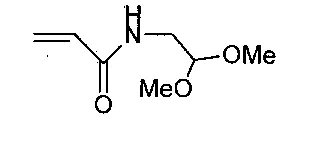 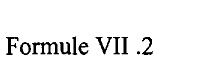 sont particulièrement préférés.
(c)les copolymères à fonctions acétals obtenus par modification chimique de polymères synthétiques ou naturels, et notamment les copolymères issus de la réaction d'un ou plusieurs aldéhydes sur le poly(vinylalcool/vinylacétate) de formule générale (X): 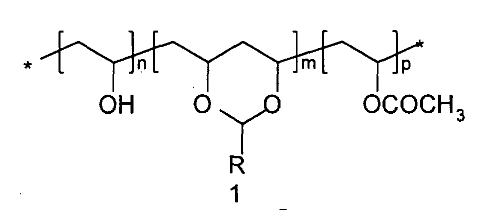 dans laquelle R a la même signification que celle donnée précédemment,
   n, m et p sont compris entre 1 et 10 000.

Ces synthèses sont connues de l'homme de l'art et décrites dans *le Précis de Matières Plastiques, J.P.Trotigon, J.Verdu, Editions Nathan, 1996.*

Les polymères à squelette non siliconé, utilisés selon l'invention sont obtenus selon les procédés classiques de polymérisation ou de modification de polymères.

Pour obtenir ces polymères, l'une des étapes du procédé d'obtention consiste, de préférence, en:
- une polycondensation,
- une ouverture d'au moins un cycle comprenant un nombre d'atomes de carbone compris entre 2 et 9 ou un cycle comprenant un nombre d'atomes de silicium compris entre 2 et 4, ces cycles pouvant inclure un ou plusieurs hétéroatomes tels que N, O, S, Si ;
- une polymérisation de monomères insaturés de type radicalaire, ionique, par transfert de groupe.

Les squelettes polymériques selon l'invention peuvent être linéaires, ramifiés, hyperbranchés ou dendritiques. Ils peuvent présenter un ou plusieurs types de motifs de répétition, et donc être des homopolymères ou des copolymères qui sont alors statistiques ou alternés ou séquencés.

Les fonctions réactives sont réparties le long des chaînes principales ou secondaires des polymères selon l'invention, éventuellement aux extrémités des chaînes dans le cas des polymères ramifiés, hyperbranchés et dendritiques.

Dans le cas où le polymère à squelette non siliconé selon l'invention est formé par un procédé de polymérisation de la liste ci-dessus, les fonctions réactives peuvent, soit être présentes sur les monomères servant de produit de départ à la polymérisation, soit être formées par réaction des monomères entre eux lors de la polymérisation, soit être apportées par au moins une étape chimique s'ajoutant à la polymérisation, par exemple une étape consistant à greffer, spécifiquement sur le polymère obtenu, des motifs moléculaires ou polymériques portant les fonctions réactives (I) à (IX) adéquates.

Pour effectuer une polycondensation, on suit par exemple, les protocoles opératoires décrits dans *Step polymerisation in principles of polymerization*, *G.* ODIAN 3 ed. Wiley interscience.

Dans le cas d'une polycondensation, les monomères utilisés comme produit de départ, sont de préférence choisis parmi les diamines ou /et diols en réaction avec diisocyanates ou diacides ou diesters, et conduisent aux polyuréthanes, polyamides, polyesters, et les aziridines et leurs dérivés conduisent aux polyalkyleneimines, tels que les polyéthylèeneimines et leurs dérivés.

Par exemple, un polyuréthane peut être obtenu par réaction des monomères isophorone diisocyanate, hexamethylenediisocyanate, methylene-biscychohexanediisocyanate, polytetramethylene glycol dihydroxyle.

Pour effectuer une ouverture de cycle comprenant un nombre d'atomes de carbone compris entre 2 et 9 ou comprenant un nombre d'atomes de silicium compris entre 2 et 4, ces cycles comprenant éventuellement un ou plusieurs hétéroatomes parmi N, O, S, Si, on suit, par exemple, les modes opératoires décrits dans *Ring opening polymerization in « Comprehensive Polymer Science ». Perg. Press vol3.*

Dans le cas d'une ouverture de cycle, les monomères utilisés comme produit de départ, pour former les polymères, sont de préférence choisis parmi les esters cycliques (lactones) et les amides cycliques (lactames), comme par exemple :

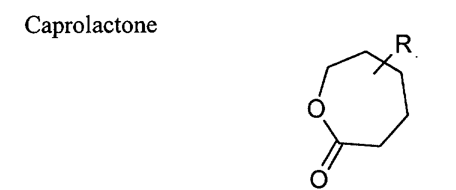

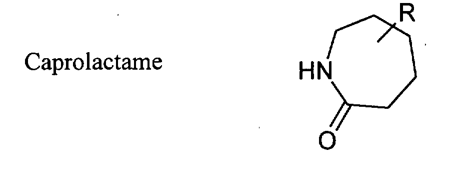

Dans le cas où le polymère selon l'invention est formé par un procédé d'obtention comprenant une étape consistant en une ouverture de cycle, les fonctions réactives peuvent, soit être présentes dans les monomères servant de produit de départ et comportant un cycle, par exemple en tant que substituant chimique présent sur les cycles, soit être formées après réaction, entre eux, de ces monomères comportant un cycle, soit être apportées par au moins une étape chimique s'ajoutant à l'étape d'ouverture de cycle, par exemple une étape séparée consistant à greffer des motifs moléculaires ou polymèriques portant les fonctions réactives (I) à (IX) adéquates.

Pour effectuer une polymérisation radicalaire ou anionique, on suit, par exemple, les modes opératoires décrits dans *Radical polymerization and anionic polymerization,* in *Principles of Polymerization*, G. ODIAN 3 ed. Wiley interscience.

Dans le cas d'une polymérisation radicalaire ou anionique, les monomères utilisés comme produit de départ, pour former les polymères, sont de préférence choisis parmi les vinyles, diènes, (méth)acrylates, (méth)acrylamides.

Dans le cas d'une polymérisation radicalaire ou anionique, le polymère est, de préférence constitué d'au moins dix unités reliées par des liaisons covalentes. Les fonctions réactives, présentes sur le polymère entrant dans la constitution des compositions conformes à l'invention, peuvent être déjà présentes sur les monomères servant de produit de départ à la réaction radicalaire, ou éventuellement être formées lors de la réaction radicalaire, ou encore par exemple, être apportées sur le polymère par une quelconque étape chimique supplémentaire.

Il est également possible d'utiliser des polymères à squelette non siliconé - naturels et polymères naturels modifiés chimiquement pour leur apporter les fonctions réactives listées ci-dessus. On citera par exemple et de façon non restrictive, les polysaccharides (cellulose, chitosane, guar et leurs dérivés), les polypeptides (acide polyaspartique, polylysine et leurs dérivés). Dans ce cas; ces polymères à squelette non siliconé présentent naturellement ou après modification des fonctions hydroxy, amine, acide carboxylique, thiol, aldéhyde, époxy dont on utilise la réactivité telle quelle dans la composition (par exemple avec les polymères portant des fonctions époxy) ou pour apporter les fonctions chimiques listées ci-dessus.

A titre d'exemple, on peut modifier le polymère comme suit:

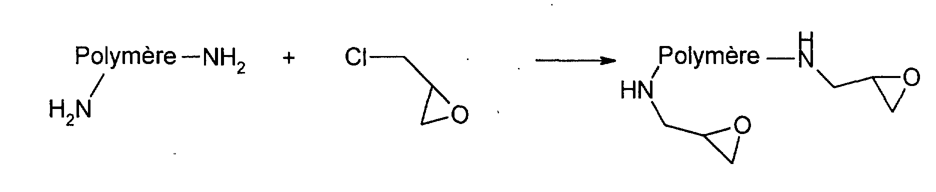

Afin de déterminer le module d'Young, on utilise une composition contenant un polymère conforme à l'invention en quantité adaptée pour obtenir, dans une matrice en téflon, un film sec d'épaisseur de 500 ± 50 µm. Le séchage est poursuivi jusqu'à ce que le poids du film n'évolue plus.

Pour mesurer le module d'Young, on effectue des essais de traction. Le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de 22 ± 2 °C et un taux d'humidité relative de 50 ± 5 %.

Les éprouvettes sont étirées à la vitesse de 20mm/mn et la distance entre les mors est de 50 ± 1 mm.

Le procédé conforme à l'invention comprend l'application sur les cheveux d'une composition cosmétique selon l'invention.

Avantageusement, il comprend les opérations supplémentaires consistant à provoquer une modification de pH et/ou, une élévation de température et/ou procéder à l'ajout d'un ou plusieurs additifs et/ou rincer.

Selon un mode de réalisation de l'invention, on applique une composition de soin, de coloration, de déformation permanente, de maquillage du cheveu, de fixation et/ou de maintien de la coiffure, avant d'appliquer une composition conforme à l'invention.

Dans les compositions conformes à l'invention, le ou les polymères à squelette non siliconé, à fonctions réactives sont, de préférence, présents à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

Conformément à l'invention, la composition contient avantageusement, en outre, des additifs cosmétiques conventionnels choisis parmi les polymères fixants, les épaississants, les tensioactifs anioniques, non ioniques, cationiques ou amphotères, les parfums, les conservateurs, les filtres solaires, les protéines, les vitamines, les provitamines, les polymères non fixants anioniques, non ioniques, cationiques ou amphotères, les huiles minérales, végétales ou synthétiques, les céramides, les pseudocéramides, les silicones volatiles ou non, linéaires ou cycliques, modifiées ou non, des agents régulateurs de pH, des oxydants, des réducteurs, des inhibiteurs, des catalyseurs et tout autre additif classiquement utilisé dans les compositions cosmétiques destinées à être appliquées sur les cheveux.

Le milieu cosmétiquement acceptable est, de préférence, constitué par de l'eau et/ou un ou plusieurs solvants cosmétiquement acceptables tels que des alcools, les esters, les cétones ou les silicones volatiles cycliques ou des mélanges eau-solvant(s), ces solvants étant de préférence des alcools en C₁-C₄.

Lorsque la composition selon l'invention est conditionnée dans un dispositif aérosol, elle comprend au moins un agent propulseur, qui peut être choisi parmi les hydrocarbures volatils tels que le n-butane, le propane, l'isobutane, le pentane, les hydrocarbures halogénés et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention peuvent être appliquées sur des cheveux secs ou humides.

L'invention va être plus complètement illustrée à l'aide de l'exemple non limitatif suivant.
m.a. signifie matière active.

### Exemple :

On réalise la composition suivante :

| | |
|---|---|
| Poly(glycidyl méthacrylate)⁽¹⁾ | 20 g (2 % m.a.) |
| Ethylènediamine | 5g |
| Ammoniaque à 22 % de NH₃ | qs pH = 9 |
| Méthyléthylcétone | 20 g |
| Eau | qsp 100 |

| | |
|---|---|
| (1) en solution à 10 % en poids dans la méthyléthyl cétone, commercialisé par Polysciences, Inc. | |

Le module d'Young du film issu de la composition est d'environ 800 Mpa.
La composition appliquée sur cheveux et séchée leur confère un gainage rigide résistant aux shampooings.

## Revendications

1. Composition cosmétique, notamment capillaire, comprenant, dans un milieu cosmétiquement acceptable, au moins un polymère à squelette non siliconé, comprenant au moins deux fonctions chimiques réactives, identiques ou différentes, non photoactivables, **caractérisée par le fait que**:
(i) un film obtenu par séchage de la composition à température ambiante (22 ± 2°C) et à un taux d'humidité relative de 50 % ± 5%, présente un module d'Young compris entre 100 et 2000 MPa, bornes incluses, mesuré à une épaisseur de 0,5 mm et à une traction de 20 mm/min,
(ii) la ou les fonctions réactives est (sont) choisie(s) parmi les groupements monovalents ou divalents ci-après :
- époxy,
- anhydride,
- chlorure d'acide,
- éthylène-imino,
- aldéhyde,
- acétal ou hémi-acétal,
- aminal ou hémi-aminal,
- cétone, alpha-halocétone ou alpha-hydroxycétone,
- lactone ou thiolactone,
- isocyanate,
- thiocyanate,
- ester de N-hydroxysuccinimide,
- imide,
- imine,
- imidate,
- oxazoline, oxazolinium, oxazine ou oxazinium,
- pyridyl-thio,
- thiosulfate,
- acétoalkylate répondant à la formule : -OCO-A'-COCH₃,
A' représentant une liaison, un groupement alkylène, linéaire ou ramifiée, comprenant de 1 à 5 atomes de carbone,
- AX,
- ASO₂X,
. X est un groupé partant choisi parmi les halogènes, OSO₃H, SO₂CH3, SO₂C₂H₅, SO₂Tos, N(CH3)₃, OPO₃R₂, CN,
Tos représentant un groupement tosylate,
R représentant soit un atome d'hydrogène, soit un radical alkyle en C₁ à C₅,
. A est un groupe alkylène, arylène, alralkylène comportant entre 1 et 22 atomes de carbone, éventuellement interrompu par un ou plusieurs cycles insaturés, et comportant, éventuellement, un ou plusieurs hétéroatomes, tels que N, S, O,
(iii) le polymère à squelette non siliconé, comprenant au moins deux fonctions chimiques réactives étant autre que le copolymère vinylbutyral/vinylalcool/vinylacétate.

2. Composition selon la revendications 1, **caractérisée par le fait que** le polymère à squelette non siliconé, contenant au moins deux fonctions réactives contient moins de 50 % en nombre de fonctions ester d'acide carboxylique par rapport au nombre total des fonctions chimiques réactives.

3. Composition selon la revendication 1, **caractérisée par le fait que** X représente un halogène choisi parmi brome, le chlore, l'iode et le fluor.

4. Composition selon la revendication 1, **caractérisée par le fait que** le groupement époxy est monovalent et répond à la formule (I): 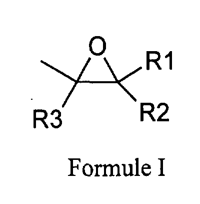 dans laquelle R₁ , R₂ et R₃ représentent, indépendamment les uns des autres :
- un atome d'hydrogène,
- un groupement alkyle, linéaire ou ramifié, comprenant 1 à 20 atomes de carbone, éventuellement interrompu par un ou plusieurs hétéroatome(s) choisi parmi O, N, S, Si et F, et éventuellement substitué par un ou plusieurs radicaux hydroxy ou amino,
- un groupement aryle comprenant entre 6 à 22 atomes de carbone ou
- un groupement aralkyle, le groupement alkyle comprenant entre 1 à 20 atomes de carbone,
- un hétérocycle de 5 à 7 chaînons.

5. Composition selon la revendication 1, **caractérisée par le fait que** le groupement anhydride d'acide carboxylique est monovalent et répond à la formule (II) : 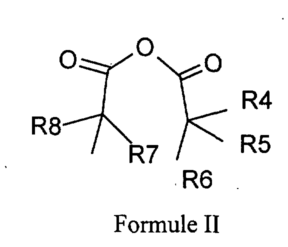 dans laquelle R ₄ , R ₅ , R ₆ , R ₇ et R ₈ ont, indépendamment les uns des autres, les mêmes significations que celles données pour R₁ , R₂ et R₃ dans la formule (I).

6. Composition selon la revendication 1, **caractérisée par le fait que** le groupement anhydride d'acide carboxylique est monovalent et répond à la formule (III) : 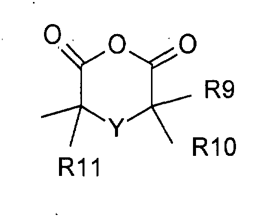 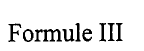 dans laquelle Y représente :
- une liaison,
- un hétéroatome choisi parmi O, N, S, Si et F,
- un radical alkyle ou alkylène, substitué ou non par un ou plusieurs radicaux hydroxy ou amino, contenant entre 1 et 5 atomes de carbone,
- un radical ralkylène, contenant entre 7 et 10 atomes de carbone ou
- un radical polydiméthylsiloxane contenant entre 1 et 6 atomes de silicium, et
R ₉ , R ₁₀ et R ₁₁ ont, indépendamment les uns des autres, les mêmes significations que celles données pour R ₁ , R ₂ et R ₃ dans la formule (I).

7. Composition selon la revendication 1, **caractérisée par le fait que** le groupement acétoalkylate est inclus dans un groupe répondant à la formule (IV) :
- R'₁-OCO-A'-COCH₃ Formule IV
dans laquelle R'₁ est obtenu par élimination de l'un quelconque des atomes d'hydrogène du radical R₁ défini dans la formule (I) et A' a la signification indiquée dans la revendication 1.

8. Composition selon la revendication 1, **caractérisée par le fait que** le groupement chlorure d'acide est inclus dans un groupe répondant à la formule (V) :
-R'₁-COCl Formule V
dans laquelle R'₁ a la même signification que dans la formule (IV).

9. Composition selon la revendication 1, **caractérisée par le fait que** le groupement isocyanate est inclus dans un groupe répondant à la formule (VI) :
-R'₁-NCO Formule VI
dans laquelle R'₁ a la même signification que dans la formule (IV).

10. Composition selon la revendication 1, **caractérisée par le fait que** le groupement acétal est monovalent et inclus dans l'une au moins des formules (VII), (VIII) ou (IX) : 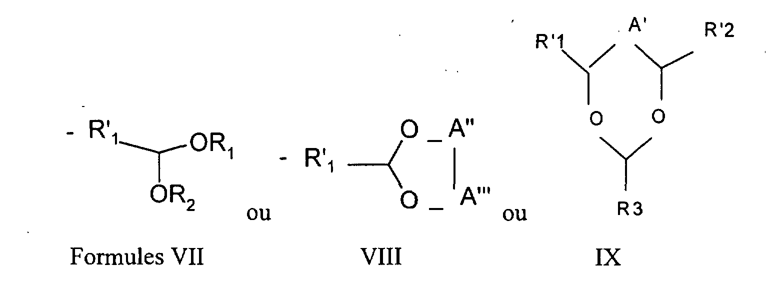 dans laquelle :
R ₁ , R ₂ et R ₃ ont la signification indiquée pour la formule (I),
R'₁ et R'₂ sont obtenus par élimination de l'un quelconque des atomes d'hydrogène du radical R₁ ou R₂ défini dans la formule (I)
A" et A"' sont identiques ou différents et représentent un groupement alkyle ou alkylène, linéaire ou ramifiée, comprenant 1 à 5 atomes de carbone, éventuellement interrompu par un ou plusieurs hétéroatome(s) choisi parmi O, N, S, Si, F, et éventuellement substitué par un ou plusieurs radicaux hydroxy ou amino.

11. Composition selon l'une quelconque des revendications 1 à 10, **caractérisée par le fait que** le polymère à squelette non siliconé, à fonction réactive est obtenu par un procédé dont l'une des étapes consiste en :
- une polycondensation,
- une ouverture d'au moins un cycle comprenant un nombre d'atomes de carbone compris entre 2 et 9 ou un cycle comprenant un nombre d'atomes de silicium compris entre 2 et 4, ces cycles pouvant inclure un ou plusieurs hétéroatomes tels que N, O, S, Si;
- une polymérisation de monomères insaturés de type radicalaire, ionique, par transfert de groupe.

12. Composition selon l'une quelconque des revendications précédentes, **caractérisée par le fait que** le ou les polymères à squelette non siliconé, à fonctions réactives est (sont) présent(s) à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

13. Composition selon l'une quelconque des revendications précédentes, **caractérisée par le fait qu'**elle comprend, en outre, des additifs cosmétiques conventionnels choisis parmi les polymères fixants, les épaississants, les tensioactifs anioniques, non ioniques, cationiques ou amphotères, les parfums, les conservateurs, les filtres solaires, les protéines, les vitamines, les provitamines, les polymères non fixants anioniques, non ioniques, cationiques ou amphotères, les huiles minérales, végétales ou synthétiques, les céramides, les pseudocéramides, les silicones volatiles ou non, linéaires ou cycliques, modifiées ou non, des agents régulateurs de pH, des oxydants, des réducteurs, des inhibiteurs, des catalyseurs et tout autre additif classiquement utilisé dans les compositions cosmétiques destinées à être appliquées sur les cheveux.

14. Composition selon l'une quelconque des revendications précédentes, **caractérisée par le fait que** le milieu cosmétiquement acceptable est constitué par de l'eau et/ou un ou plusieurs solvants cosmétiquement acceptables tels que des alcools ou les silicones volatiles cycliques ou des mélanges eau-solvant(s), ces solvants étant de préférence des alcools en C₁-C₄.

15. Dispositif aérosol comprenant une composition selon l'une quelconque des revendications précédentes, ainsi qu'au moins un agent propulseur.

16. Procédé de traitement cosmétique, **caractérisé par le fait qu'**il comprend l'application d'une composition selon l'une quelconque des revendications 1 à 14.

17. Procédé selon la revendication 16, **caractérisé par le fait qu'**on applique une composition de soin, de coloration, de déformation permanente, de maquillage du cheveu, de fixation et/ou de maintien de la coiffure, avant d'appliquer une composition selon l'une quelconque des revendications 1 à 14.

18. Utilisation d'une composition selon l'une quelconque des revendications 1 à 14, pour former un gainage rigide sur les cheveux.
